(19) Europäisches Patentamt
European Patent Office
Office européen des brevets

(11) **EP 2 211 803 B1**

(12) **EUROPÄISCHE PATENTSCHRIFT**

(45) Veröffentlichungstag und Bekanntmachung des Hinweises auf die Patenterteilung:
**09.12.2015 Patentblatt 2015/50**

(51) Int Cl.:
***A61F 9/008*** *(2006.01)*

(21) Anmeldenummer: **08847230.3**

(22) Anmeldetag: **27.10.2008**

(86) Internationale Anmeldenummer:
**PCT/EP2008/009076**

(87) Internationale Veröffentlichungsnummer:
**WO 2009/059711 (14.05.2009 Gazette 2009/20)**

(54) **BEHANDLUNGSVORRICHTUNG ZUR OPERATIVEN FEHLSICHTIGKEITSKORREKTUR EINES AUGES UND VERFAHREN ZUM ERZEUGEN VON STEUERDATEN DAFÜR**

TREATMENT DEVICE FOR SURGICALLY CORRECTING AMETROPIA OF AN EYE AND METHOD FOR CREATING CONTROL DATA THEREFORE

DISPOSITIF DE TRAITEMENT PERMETTANT LA CORRECTION CHIRURGICALE DE L'AMÉTROPIE D'UN OEIL ET PROCÉDÉ DE GÉNÉRATION DE DONNÉES DE COMMANDE ASSOCIÉES

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **08.11.2007 DE 102007053283**

(43) Veröffentlichungstag der Anmeldung:
**04.08.2010 Patentblatt 2010/31**

(73) Patentinhaber: **Carl Zeiss Meditec AG**
**07745 Jena (DE)**

(72) Erfinder:
• **BISCHOFF, Mark**
**07749 Jena (DE)**
• **STOBRAWA, Gregor**
**07743 Jena (DE)**
• **BISSMANN, Wilfried**
**07749 Jena (DE)**

(74) Vertreter: **Geyer, Fehners & Partner Patentanwälte**
**Perhamerstrasse 31**
**80687 München (DE)**

(56) Entgegenhaltungen:
WO-A2-2008/125173    US-A1- 2003 212 387
US-A1- 2006 155 265    US-B1- 6 325 792

**Beschreibung**

Die Erfindung bezieht sich auf eine Behandlungsvorrichtung zur operativen Hyperopiekorrektur am Auge, wobei die Behandlungsvorrichtung eine von einer Steuereinrichtung gesteuerte Lasereinrichtung aufweist, welche durch Einstrahlen von Laserstrahlung Hornhaut-Gewebe trennt, wobei die Steuereinrichtung ausgebildet ist, die Lasereinrichtung zur Abgabe der Laserstrahlung in die Hornhaut so anzusteuern, daß damit ein lentikelförmiges Volumen in der Hornhaut isoliert wird, dessen Entfernung aus der Hornhaut die gewünschte Hyperopiekorrektur bewirkt, wobei die Steuereinrichtung bei der Ansteuerung der Lasereinrichtung das lentikelförmige Volumen so vorgibt, daß es eine posteriore Fläche und eine anteriore Fläche hat, wobei letztere zumindest in einer Hauptebene schwächer gekrümmt ist, als die posteriore Fläche, und deren Ränder über eine Randfläche verbunden sind, wobei die Schnittkurve aus der Randfläche und einer Ebene, in der die Sehachse enthalten ist, quer zur Sehachse eine Breite hat, die größer als diejenige ist, welche in der gleichen Projektionsebene eine gerade Strecke hätte, die am Rand der posterioren oder der anterioren Fläche auf der jeweiligen Fläche senkrecht steht und die anteriore Fläche mit der posterioren Fläche oder mit deren gedachter Fortsetzung verbindet.

Die Erfindung bezieht sich weiter auf ein Verfahren zum Erzeugen von Steuerdaten für eine Lasereinrichtung einer Behandlungsvorrichtung zur operativen Hyperopiekeitskorrektur am Auge, welche durch Einstrahlen von Laserstrahlung Hornhaut-Gewebe trennt, wobei die Steuerdaten im Betrieb die Lasereinrichtung zur Abgabe der Laserstrahlung in die Hornhaut so ansteuern, daß damit ein lentikelförmiges Volumen in der Hornhaut isoliert wird, dessen Entfernung aus der Hornhaut die gewünschte Hyperopiekorrektur bewirkt, wobei die Steuerdaten das lentikelförmige Volumen so vorgeben, daß es eine posteriore Fläche und eine anteriore Fläche hat, wobei letztere zumindest in einer Hauptebene schwächer gekrümmt ist, als die posteriore Fläche, und deren Ränder über eine Randfläche verbunden sind, wobei die Schnittkurve aus der Randfläche und einer Ebene, in der die Sehachse enthalten ist, quer zur Sehachse eine Breite hat, die größer als diejenige ist, welche in der gleichen Projektionsebene eine gerade Strecke hätte, die am Rand der posterioren oder der anterioren Fläche auf der jeweiligen Fläche senkrecht steht und die anteriore Fläche mit der posterioren Fläche oder mit deren gedachter Fortsetzung verbindet.

Eine solche Vorrichtung und ein solches Verfahren sind aus der US 6,325,792 B1 bekannt.

Der klassische Weg zur Korrektur der Fehlsichtigkeit des menschlichen Auges ist die Brille. Mittlerweile wird jedoch auch vermehrt refraktive Chirurgie eingesetzt, die durch Veränderung der Augenhornhaut eine Fehlsichtigkeitskorrektur bewirkt Ziel der Operationsmethoden ist es dabei, die Hornhaut gezielt zu verändern, um so die Lichtbrechung zu beeinflussen. Hierfür sind unterschiedliche Operationsmethoden bekannt. Am verbreitesten ist gegenwärtig die sogenannte Laser-Insitu-Keratomileusis, die auch LASIK abgekürzt wird. Dabei wird zuerst eine Hornhautlamelle von der Hornhautoberfläche einseitig gelöst und zur Seite geklappt. Das Lösen dieser Lamelle kann mittels eines mechanischen Mikrokeratoms erfolgen, oder auch mittels eines sogenannten Laserkeratoms, wie es z.B. von Intralase Corp. Irvine, USA, vertrieben wird. Nachdem die Lamelle gelöst und zur Seite geklappt wurde, ist bei der LASIK-Operation die Anwendung eines Excimer-Lasers vorgesehen, der das derart freigelegte Hornhautgewebe durch Ablation abträgt. Nachdem auf diese Art und Weise in der Hornhaut liegendes Volumen verdampft wurde, wird die Hornhautlamelle wieder auf den ursprünglichen Platz zurückgeklappt.

Dieses Vorgehen ist z. B. Gegenstand der US 2006/0155265 A1 oder der US 2003/0212387 A1, wobei letztere Druckschrift vorschlägt, ein Reservoir zur Aufnahme von Gasen, Fluiden oder Abbauprodukten vorzusehen, die bei der Ablation entstehen.

Die Anwendung eines Laserkeratoms zum Freilegen der Lamelle ist vorteilhaft, da die Infektionsgefahr dadurch verringert und die Schnittqualität vergrößert ist. Insbesondere kann die Lamelle mit sehr viel konstanterer Dicke hergestellt werden. Auch ist der Schnitt potentiell glatter, was spätere optische Störungen durch diese auch nach der Operation verbleibende Grenzefläche mindert.

Bei der Erzeugung einer Schnittfläche in der Hornhaut durch Laserstrahlung wird ins Gewebe üblicherweise gepulste Laserstrahlung eingebracht, wobei die Pulslänge in der Regel unter 1 ps liegt. Dadurch wird die zur Auslösung eines optischen Durchbruchs nötige Leistungsdichte für den jeweiligen Puls auf ein enges räumliches Gebiet begrenzt. Die US 5984916 zeigt diesbezüglich deutlich, daß der räumliche Bereich des optischen Durchbruches (in diesem Fall der erzeugten Wechselwirkung) stark von der Pulsdauer abhängt. Eine hohe Fokussierung des Laserstrahls in Kombination mit den erwähnten kurzen Pulsen erlaubt es damit, den optischen Durchbruch punktgenau in der Hornhaut einzusetzen. Zur Schnitterzeugung wird eine Serie optischer Durchbrüche an vorbestimmten Stellen so erzeugt, daß dadurch die Schnittfläche ausgebildet wird. Beim erwähnten Laserkeratom bildet die Schnittfläche die vor dem Einsatz der Laserablation abzuklappende Lamelle.

Bei der herkömmlichen LASIK-Methode wird freigelegtes Hornhautgewebe verdampft, was auch als "Schleifen" der Hornhaut mittels Laserstrahlung bezeichnet wird. Die Volumenentfernung, die für eine Fehlsichtigkeitskorrektur notwendig ist, wird dabei für jedes Flächenelement der freigelegten Hornhaut durch die Zahl der Laserpulse und deren Energie eingestellt. Je nach Zahl und Energie der Laserpulse erfolgt unterschiedlich viel Materialabtrag.

In jüngster Zeit wurde das eingangs erwähnte Operationsverfahren beschrieben und in ersten Erprobungen untersucht. Mittels Laserstrahlung wird ein Volumen in der Hornhaut isoliert und dann das dieses Volumen bildende Gewebestück entnommen. Da auch hier i.d.R. gepulste Laserstrahlung verwendet wird, spricht man von Femtosekemden-Lentikel-Extraktion oder kurz FLEx. Das Volumen wird als Lentikel bezeichnet.

Erfahrungswerte, die zum Schleifen der Hornhaut mittels Ablationslaserstrahlung tauglich sind, können nun für das FLEx-Verfahren der refraktiven Augenchirurgie, bei dem das aus der Hornhaut zu entfernende Volumen nicht durch Ablation freigelegten Hornhautgewebes abgetragen wird, sondern in der Hornhaut durch eine dreidimensionale Schnittfläche isoliert wird und somit entnehmbar gemacht, nicht verwendet werden, da die Ansätze, Verdampfen von zu entfernenden Material einerseits und Entnahme eines isolierten Volumens andererseits, zu unterschiedlich sind. Dies gilt ganz besonders für die Wahl der das zu entnehmenden Volumen begrenzenden Schnittfläche, da es eine solche bei der herkömmlichen LASIK-Operation gar nicht gibt. Auch sind die Heilungsprozesse nach der Operation andere, da die Flächen andere Oberflächenstrukturen haben.

Die manuelle Entfernung erfordert eine gewisse mechanische Stabilität des Gewebestücks, welche neben der beabsichtigten refraktiven Wirkung eine weitere Randbedingung darstellt. Unter diesem Aspekt sollte deshalb das Gewebestück so dick ausgeführt werden, wie es die Hornhautrestdicke, die sich nach Entnahme des Gewebestückes einstellt, gerade noch zuläßt.

Diesem Wunsch steht der Bedarf nach einer Formgebung entgegen, die es erlaubt, die refraktiven Auswirkungen der Heilungsprozesse zu minimieren oder zumindest vorhersagbar zu gestalten. Sind die Lentikel nämlich so geformt, daß Heilungsprozesse in einem erheblichen Maß zur Veränderung der durch die refraktiven Therapie unmittelbar hervorgerufenen Korrektur beitragen, z. B. durch eine Hornhautverdickung, so würde dies von den Patienten als ungünstig empfunden. Solche negativen Einflüsse von Heilungsprozessen werden als Regression bezeichnet Es ist erforderlich, Heilungsprozesse möglichst ohne Regression, also ohne refraktive Veränderungen ablaufen zu lassen; mit anderen Worten: die refraktive Wirkung soll möglichst rasch im erwünschten Umfang eintreten und dann für alle Zeiten konstant bleiben. Nicht immer ist dies aber möglich, weshalb die mit Heilungsprozessen verbundene Regression oftmals in die Planung einer refraktiven Therapie einbezogen werden muß. Aufgrund physiologischer Eigenschaften des menschlichen Auges ist es zu erwarten, daß hyperope Korrekturen mittels FLEx-Methode von derartigen Heilungsprozessen generell stärker beeinflußbar sind. Es besteht daher der Wunsch, diese refraktiven Veränderungen im Zuge der Heilungsprozesse, wenn sie denn schon nicht vermieden werden können, so doch zumindest möglichst gering und vorhersagbar zu gestalten.

Hier hilft der Stand der Technik bei Hyperopiekorrekturen nicht weiter, da die bislang bekannten Schnitt- und Lentikel-Formen sich auf Myopiekorrekturen beziehen.

Insgesamt bestehen somit bei der refraktiven Fehlsichtigkeitskorrektur mittels FLEX wie es z. B. in der WO 2008/125173 A2 geschildert ist, in vielen Bereichen andere und z.T. auch neue Randbedingungen als bei der herkömmlichen LASIK-Methode.

Es ist deshalb Aufgabe der vorliegenden Erfindung, eine Behandlungsvorrichtung bzw. ein Verfahren der eingangs genannten Art so auszubilden, daß die Schnittflächen für das Lentikel sowohl für eine sichere Entnahme als auch für den Heilungsprozeß günstig sind.

Diese Aufgabe wird erfindungsgemäß gelöst mit einer Behandlungsvorrichtung zur operativen Hyperopiekorrektur mit den Merkmalen des Anspruchs 1.

Diese Aufgabe wird weiter gelöst mit einem Verfahren zum Erzeugen von Steuerdaten für eine Lasereinrichtung einer Behandlungsvorrichtung zur operativen Hyperopiekorrektur am Auge, mit den Merkmalen des Anspruchs 2.

Die Erfindung geht von der Erkenntnis aus, daß postoperative (Regressions-)Probleme beim FLEX-Verfahren davon herrühren, daß die gelöste und wiederaufgelegte Hornhautlamelle sich am Rand des entnommenen Volumens ungünstig einfügt bzw. nicht glatt aufliegen kann. Zur Vermeidung solcher Regressionsprobleme schafft die Erfindung eine breite Randzone, so daß für die dort auf die posteriore Schnittfläche, die zur Isolierung des lentikelförmigen Volumens geschaffen wurde, übergehende Hornhautlamelle eine Übergangszone geschaffen ist, die so gut wie keine Regression zur Folge hat. Wenn diese Übergangszone vorzugsweise auch außerhalb des optisch wirksamen Bereichs liegt, also außerhalb der dunkeladaptierten Pupille des Auges, ist die Gefahr für weitere unerwünschte Nebenwirkungen nochmals verringert. Vorzugsweise hat die Übergangszone eine Breite zwischen 0,1 und 1 mm.

Es zeigte sich weiter, daß die Regressionen besonders gut gemindert sind, wenn die Randfläche möglichst senkrecht in die anteriore Fläche mündet. Ein solcher Verlauf ist primär überraschend, da bei einer solch senkrechten Mündung für die darüberliegende Hornhautlamelle auch eine senkrecht von der Hornhautvorderfläche wegführende Stufe gegeben sein kann. Diese senkrechte Mündung erweist sich jedoch hinsichtlich der Regression als unproblematisch, wenn die Randfläche unterhalb dieser senkrechten Mündung einen zweiten, stärker zur Sehachse hingeneigten Abschnitt aufweist. Durch diese Struktur hat das lentikelförmige Volumen eine Randdicke, die vorzugsweise zwischen 5 und 10 μm liegt. Es zeigte sich, daß eine solche Randdicke hinsichtlich der Regression unproblematisch ist, zugleich aber eine bessere Entnehmbarkeit des Gewebestückes gewährleistet, da durch die Randdicke mit ausreichender Sicherheit vermieden ist, daß beim Abnehmen des Gewebestückes Partikel am Rand abreißen. Solche Partikel beeinflussen das Auflegen der Hornhautlamelle nach Entnahme des Gewebes sehr viel stärker, als es die vergleichsweise geringer Randdicke des Gewebestückes und damit der kurze senkrecht mündende Abschnitt der Randfläche tut. Diese Ausgestaltung der Erfindung nimmt also eine apriori als negativ erscheinende Randstruktur und erzielt damit im Ergebnis eine geringere Regression, d.h. ein besseres Einwachsverhalten, als ein eigentlich zu erwartender möglichst schmaler Rand.

Der zweite Abschnitt der Randfläche, der stärker zur Sehachse hingeneigt ist, kann auf viele Arten ausgebildet werden. Beispielsweise ist ein gerader, in einem Winkel zum ersten Abschnitt stehender Verlauf möglich, der in einem Winkel von 80° bis 100° zur Richtung der Sehachse liegt. Der zweite Abschnitt der Randfläche kann dann als Anfasung verstanden werden. Es sind aber auch gekrümmte Verläufe möglich, beispielsweise ein bezogen auf den Durchtrittspunkt der Sehachse konkaver Verlauf, d.h. eine Krümmung des zweiten Abschnittes zur Sehachse hin. In diesen Fall ist der zweite Abschnitt dann beispielsweise in Form einer Abrundung ausgeführt.

Im Rahmen der Erfindung sind verschiedenste Geometrien für den Rand tauglich, die alle das Regressionsverhalten positiv beeinflussen, indem sie die geschilderte breite Übergangszone bereitstellen.

In einer Weiterbildung der Erfindung wird die Randstruktur mit einer Bemessungsregel für das Hornhautvolumen kombiniert, welche den Krümmungsradius definiert, den die Hornhaut nach der Entnahme des Volumens hat. Diese Fortbildung der Erfindung erlaubt also nicht nur eine regressionsoptimierte Randstruktur, sondern auch eine analytische Berechnung der posterioren und der anterioren Fläche.

Die Beschreibung der Hornhautvorderseitenkrümmung nach der Korrektur geht dabei von Fehlsichtigkeitsdaten aus, welche die Brechkraft $B_{BR}$ einer für die Fehlsichtigkeitskorrektur tauglichen Brille angeben, die in einem Abstand $d_{HS}$ vor dem Hornhautscheitel liegen muß, um die gewünschte Fehlsichtigkeitskorrektur zu erreichen. Das Bestimmen dieser Parameter ist gängiger Standard in der Augenheilkunde und ermöglicht die Verwendung vorhandener Meßgeräte. Selbstverständlich können die dabei verwendeten Meßdaten auch Astigmatismusfehler oder Fehler höherer Aberrationsordnungen wiedergeben, so daß die Gleichung zur Beschreibung des Krümmungsradius, der um das Volumen verminderten Hornhaut dann entsprechende Winkelparameter (bezogen auf zylindrische Koordinaten) hat, wie nachfolgende Gleichung (1) der Figurenbeschreibung wiedergibt.

Es ist deshalb eine Weiterbildung der erfindungsgemäßen Vorrichtung bzw. des erfindungsgemäßen Verfahrens bevorzugt, bei der die anteriore Fläche in einem konstantem Abstand $d_F$ zur Hornhautvorderfläche liegt und die posteriore Fläche gekrümmt ist und einen Krümmungsradius $R_L = R_{CV}^* - d_F$ hat, wobei $R_{CV}^*$ folgender Gleichung genügt $R_{CV}^* = 1 / ( ( 1/R_{CV}) + B_{BR} / ((n_c-1) \cdot (1 - d_{HS} \cdot B_{BR}))) + F$, und $R_{CV}$ der Krümmungsradius der Hornhaut vor Entfernung des Volumens, $n_c$ die Brechkraft des Materials der Hornhaut, F ein Korrektur-Faktor ist, $B_{BR}$ die Brechkraft einer für die Fehlsichtigkeitskorrektur tauglichen Brille, sowie $d_{HS}$ der Abstand ist, in dem die Brille mit der Brechkraft $B_{BR}$ vor dem Hornhautscheitel liegen müßte, um die gewünschte Fehlsichtigkeitskorrektur mittels der Brille zu erreichen.

Der Korrektur-Faktor F stellt ein Maß für die optische Wirkung der Dickenabnahme der Augenhornhaut auf der Sehachse dar, welche sich durch die Entfernung des Volumens ergibt. In einer vereinfachten Berechnung kann der Faktor F=0 gesetzt werden. In einer genaueren Berechnung kann F wie folgt berechnet werden: $F = (1 - 1/n_c) \cdot (d_C^* - d_C)$, wobei $d_c$ bzw. $d_c^*$ die Dicke der Hornhaut vor bzw. nach Entfernung des Volumens bezeichnet und der Radius $R_{CV}^*$ iterativ berechenbar ist, indem bei jedem Iterationsschritt aus der Differenz $(R_{CV}^* - R_{CV})$ auf eine Dickenänderung $(d_C^* - d_C)$ geschlossen wird und das entsprechende daraus erhaltene Ergebnis für die Dickenänderung bei der Berechnung von $R_{CV}^*$ im nächsten Iterationsschritt angewendet wird. Die iterative Berechnung für F kann beispielsweise abgebrochen werden, wenn zwischen zwei Iterationsschritten für F nur noch ein Unterschied besteht, der kleiner als ein bestimmter Grenzwert ist.

Die in der Weiterbildung vorgesehene Ausgestaltung der posterioren Fläche mit einer Krümmung, welche auf die der Hornhautvorderfläche nach der Entfernung des Volumens Bezug nimmt, erlaubt eine besonders einfache Definition der das Volumen begrenzenden Flächen, da die anteriore Fläche nun in einem konstantem Abstand zur Hornhautvorderfläche liegt und die optische Korrektur durch die Formgebung der posterioren Fläche bewirkt wird. Merklicher Rechenaufwand entsteht dann nur noch für die Definition der posterioren Teilfläche, nicht hingegen für die anteriore Teilfläche. Weiter zeigt sich auch, daß bei einem derartigen Ansatz zugleich eine einfache analytische Beschreibung der posterioren Teilfläche möglich ist.

Das erfindungsgemäße Verfahren zum Vorbereiten der Steuerdaten kann ohne menschliche Mitwirkung durchgeführt werden. Insbesondere kann es von einem Computer ausgeführt werden, der aus entsprechenden Vorgaben, beispielsweise aus Meßdaten des Auges die Steuerdaten ermittelt. Vor allem ist bei der Ermittlung der Steuerdaten die Mitwirkung eines Arztes in keiner Weise erforderlich, da mit der Ermittlung der Steuerdaten noch kein therapeutischer Eingriff verbunden ist. Dieser findet erst bei der Anwendung der zuvor ermittelten Steuerdaten statt.

Soweit in dieser Beschreibung Verfahrensschritte geschildert werden, ist in der erfindungsgemäßen Vorrichtung ein Steuergerät vorgesehen, daß für die Ausführung der Verfahrensschritte beim Betrieb der Vorrichtung Sorge trägt.

Die Erfindung wird nachfolgend unter Bezugnahme auf die Zeichnungen beispielshalber noch näher erläutert. In den Zeichnungen zeigt:

Fig. 1        eine Schemadarstellung einer Behandlungsvorrichtung bzw. eines Behandlungsgerätes zur Fehlsichtigkeitskorrektur,

Fig. 1a      eine Schemadarstellung hinsichtlich des Aufbaus des Behandlungsgerätes der Fig. 1,

Fig. 2      eine Prinzipdarstellung zur Einbringung gepulster Laserstrahlung in das Auge bei der Fehlsichtigkeitskorrektur mit dem Behandlungsgerät der Fig. 1,

Fig. 3      eine weitere Schemadarstellung des Behandlungsgerätes der Fig. 1,

Fig. 4      in Teilfiguren (a), (b) und (c) schematische Schnittdarstellungen zur Verdeutlichung des Korrekturbedarfes am menschlichen Auge bei Fehlsichtigkeit,

Fig. 5      eine schematische Schnittdarstellung durch die Augenhornhaut mit Darstellung eines zur Fehlsichtigkeitskorrektur zu entfernenden Volumens,

Fig. 6      ein Schnitt durch die Augenhornhaut nach Entfernung des Volumens der Fig. 5,

Fig. 7      eine Schnittdarstellung ähnlich der Fig. 5,

Fig.8      eine schematische Schnittdarstellung durch die Augenhornhaut zur Veranschaulichung der Volumenentnahme,

Fig. 9a      eine Draufsicht auf die Augenhornhaut zur Veranschaulichung der bei der Fehlsichtigkeitskorrektur erzeugten Schnittflächen,

Fig. 9b      eine Schnittdarstellung zur Draufsicht der Fig. 9a, in der das Profil eines zur Hyperopie-Korrektur entfernten Volumens veranschaulicht ist,

Fig. 10a      eine Darstellung ähnlich der Fig. 9a, hier jedoch für eine Myopie-Korrektur,

Fig. 10b      eine Darstellung ähnlich der Fig. 9a, jedoch wiederum für Myopie-Korrektur,

Fig. 11a      eine Darstellung ähnlich der Fig. 9a, jedoch mit einer anderen Schnittführung bezüglich der Entnahme des Volumens,

Fig. 11b      eine Schnittdarstellung ähnlich der Fig. 9b, jedoch für die Draufsicht der Fig. 11a,

Fig. 12a      eine Schnittdarstellung ähnlich der Fig. 9a, jedoch mit einem andersartigen Randschnitt zur Begrenzung des Volumens, welches zur Fehlsichtigkeitskorrektur entnommen wird,

Fig. 12b      eine vergrößerte Teilansicht des Randschnittes der Fig. 9a und

Fig. 12c und 12d      eine vergrößerte Randschnitt-Darstellungen ähnlich der Fig. 12b, jedoch für andere Geometrien der Randfläche.

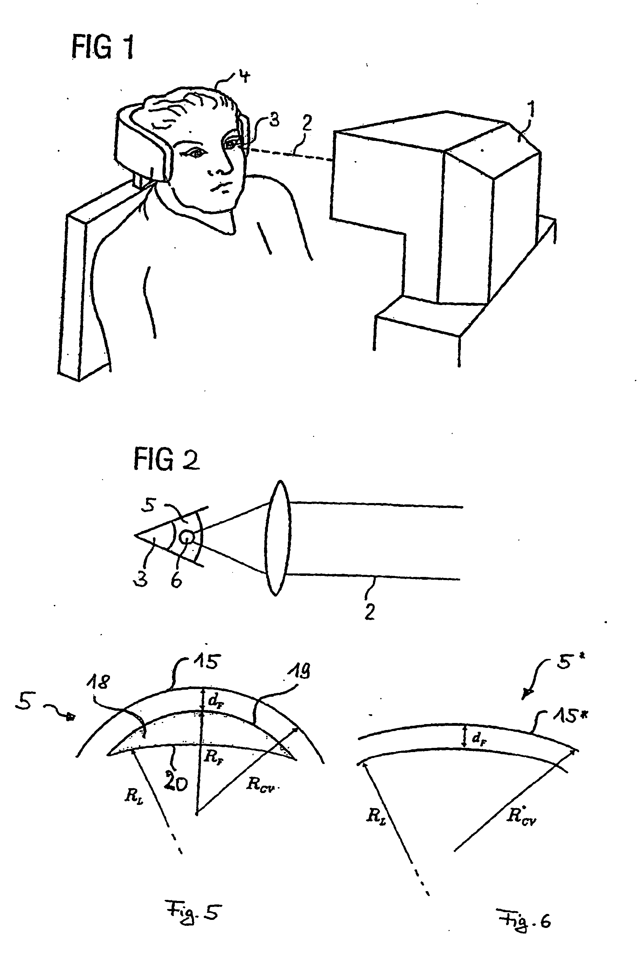

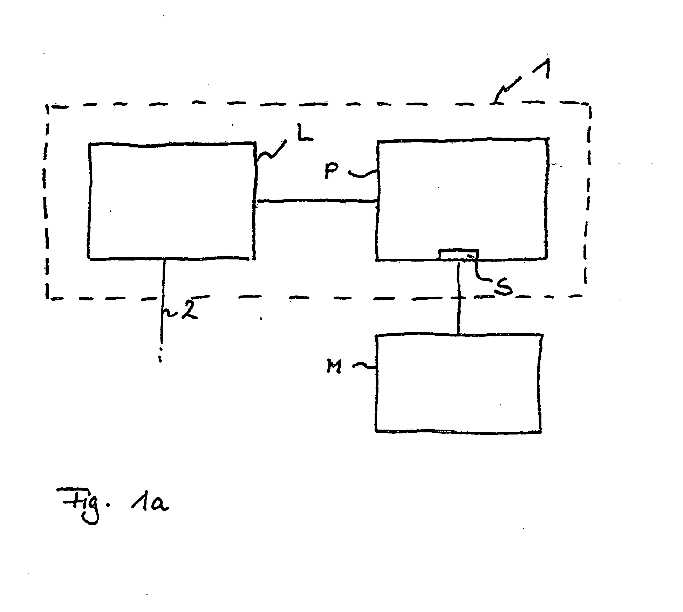

Figur 1 zeigt ein Behandlungsgerät 1 für ein augenchirurgisches Verfahren, das dem in der EP 1159986 A1 bzw. der US 5549632 beschriebenen ähnelt. Das Behandlungsgerät 1 bewirkt mittels ein Behandlungs-Laserstrahlung 2 eine Fehlsichtigkeitskorrektur an einem Auge 3 eines Patienten 4. Die Fehlsichtigkeit kann Hyperopie, Myopie, Presbyopie, Astigmatismus, gemischten Astigmatismus (Astigmatismus, bei dem in einer Richtung Hyperopie und in einer rechtwinklig dazu liegenden Richtung Myopie vorliegt), asphärische Fehler und Abberationen höherer Ordnung umfassen. Die Behandlungs-Laserstrahlung 2 wird in der beschriebenen Ausführungsform als gepulster in das Auge 3 fokussierter Laserstrahl aufgebracht. Die Pulsdauer liegt dabei z.B. im Femtosekundenbereich, und die Laserstrahlung 2 wirkt mittels nicht-linearer optischer Effekte in der Hornhaut. Der Laserstrahl weist z.B. 50 bis 800 fs kurze Laserpulse (bevorzugt 100 - 400 fs) mit einer Pulswiederholfrequenz zwischen 10 und 500 kHz auf. Die Baugruppen des Gerätes 1 werden im beschriebenen Ausführungsbeispiel von einer integrierten Steuereinheit gesteuert, die aber natürlich auch eigenständig ausgebildet sein kann.

Vor dem Einsatz des Behandlungsgerätes wird die Fehlsichtigkeit des Auges 3 mit einer oder mehreren Meßeinrichtungen vermessen.

Figur 1 a zeigt schematisch das Behandlungsgerät 1. Es weist in dieser Variante mindestens zwei Einrichtungen oder Module auf. Eine Lasereinrichtung L gibt den Laserstrahl 2 auf das Auge 3 ab. Der Betrieb der Lasereinrichtung L erfolgt dabei vollautomatisch, d.h. die Lasereinrichtung L startet auf ein entsprechendes Startsignal hin die Ablenkung des Laserstrahls 2 und erzeugt dabei Schnittflächen, die auf noch zu beschreibende Art und Weise aufgebaut sind und ein Volumen in der Augenhornhaut isolieren. Die für den Betrieb erforderlichen Steuerdaten empfängt die Lasereinrichtung L zuvor von einer Planungseinrichtung P als Steuerdatensatz über nicht näher bezeichnete Steuerleitungen. Die Übertragung findet vor dem Betrieb der Lasereinrichtung L statt. Natürlich kann Kommunikation auch drahtlos erfolgen. Alternativ zu einer direkten Kommunikation ist es auch möglich, die Planungseinheit P räumlich getrennt von der Lasereinheit L anzuordnen und einen entsprechenden Datenübertragungskanal vorzusehen.

Vorzugsweise wird der Steuerdatensatz zum Behandlungsgerät 1 übertragen und weiter vorzugsweise ist ein Betrieb der Lasereinrichtung L gesperrt, bis an der Lasereinrichtung L ein gültiger Steuerdatensatz vorliegt. Ein gültiger Steuerdatensatz kann ein Steuerdatensatz sein, der prinzipiell zur Verwendung mit der Lasereinrichtung L der Behandlungsvorrichtung 1 geeignet ist. Zusätzlich kann die Gültigkeit aber auch daran geknüpft werden, daß weitere Prüfungen bestanden werden, beispielsweise ob im Steuerdatensatz zusätzlich niedergelegte Angaben über das Behandlungsgerät 1, z. B. eine Geräteseriennummer, oder den Patienten, z. B. eine Patientenidentifikationsnummer, mit anderen Angaben übereinstimmen, die beispielsweise an der Behandlungsvorrichtung ausgelesen oder separat eingegeben wurden, sobald der Patient in der korrekten Stellung für den Betrieb der Lasereinrichtung L ist.

Die Planungseinheit P erzeugt den Steuerdatensatz, der der Lasereinheit L zur Ausführung der Operation zur Verfügung gestellt wird, aus Meßdaten und Fehlsichtigkeitsdaten, die für das zu behandelnde Auge ermittelt wurden. Sie werden der Planungseinheit P über eine Schnittstelle S zugeführt und stammen im dargestellten Ausführungsbeispiel aus einer Meßeinrichtung M, die das Auge des Patienten 4 zuvor vermessen hat. Natürlich kann die Meßeinrichtung M auf beliebige Art und Weise die entsprechenden Meß- und Fehlsichtigkeitsdaten an die Planungseinrichtung P übermitteln.

Die Übertragung kann mittels Speicherchips (z.B. per USB oder memory stick), Magnetspeichern (z.B. Disketten), per Funk (z.B. WLAN, UMTS, Bluetooth) oder drahtgebunden (Z.B. USB, Firewire, RS232, CAN-Bus, Ethernet etc.) erfolgen. Gleiches gilt natürlich hinsichtlich der Datenübertragung zwischen Planungseinrichtung P und Lasereinrichtung L.

Eine direkte Funk- oder Draht-Verbindung der Meßeinrichtung M mit der Behandlungseinrichtung 1 hinsichtlich der Datenübertragung, die in einer Variante verwendet werden kann, hat den Vorteil, daß die Verwendung falscher Meß- und Fehlsichtigkeitsdaten mit größtmöglicher Sicherheit ausgeschlossen ist. Dies gilt insbesondere dann, wenn die Überführung des Patienten von der Meßeinrichtung M bzw. den Meßeinrichtungen zur Lasereinrichtung L mittels einer (in der Figur nicht dargestellten) Lagerungseinrichtung erfolgt, die mit der Meßeinrichtung M bzw. der Lasereinrichtung L so zusammenwirkt, daß die jeweiligen Einrichtungen erkennen, ob der Patient 4 in der jeweiligen Position zum Vermessen bzw. Einbringen der Laserstrahlung 2 ist. Mit einem Verbringen des Patienten 4 von der Meßeinrichtung M zur Lasereinrichtung L kann dabei zugleich auch die Übertragung der Meß- und Fehlsichtigkeitsdaten an die Behandlungsvorrichtung 1 erfolgen.

Es ist vorzugsweise durch geeignete Mittel sichergestellt, daß die Planungseinrichtung P immer den zum Patienten 4 gehörenden Steuerdatensatz erzeugt, und eine irrtümliche Verwendung eines falschen Steuerdatensatzes für einen Patienten 4 ist so gut wie ausgeschlossen.

Die Wirkungsweise des Laserstrahls 2 ist in Figur 2 schematisch angedeutet. Der Behandlungs-Laserstrahl 2 wird mittels einer nicht näher bezeichneten Optik in die Hornhaut 5 des Auges 6 fokussiert. Dadurch entsteht in der Hornhaut 5.ein Fokus, der einen Spot 6 überdeckt und in dem die Laserstrahlungsenergiedichte so hoch ist, daß in Kombination mit der Pulslänge ein nicht-linearer Effekt im Auge auftritt. Beispielsweise kann jeder Puls der gepulsten Laserstrahlung 2 am jeweiligen Spot 6 einen optischen Durchbruch in der Augenhornhaut 5 erzeugen, welcher wiederum eine in Figur 2 schematisch angedeutete Plasmablase initiiert. Dadurch wird mittels dieses Laserpulses in der Hornhaut 5 Gewebe getrennt. Bei Entstehung einer Plasmablase umfaßt die Gewebeschichttrennung ein größeres Gebiet, als den Spot 6, welchen der Fokus der Laserstrahlung 2 überdeckt, obwohl die Bedingungen zur Erzeugung des Durchbruches nur im Fokus erreicht werden. Damit von jedem Laserpuls ein optischer Durchbruch von jedem Laserpuls erzeugt wird, muß die Energiedichte, d.h. die Fluence der Laserstrahlung oberhalb eines gewissen, pulslängenabhängigen Schwellwertes liegen. Dieser Zusammenhang ist dem Fachmann beispielsweise aus der DE 69500997 T2 bekannt.

Alternativ kann ein gewebetrennender Effekt durch die gepulste Laserstrahlung auch dadurch erzeugt werden, daß mehrere Laserstrahlungspulse im einen Bereich abgegeben werden, wobei sich für mehrere Laserstrahlungspulse die Spots 6 überlappen. Es wirken dann mehrere Laserstrahlungspulse zusammen, um einen gewebetrennenden Effekt zu erreichen.

Die Art der Gewebetrennung, die das Behandlungsgerät 1 einsetzt, ist jedoch für die nachfolgende Beschreibung nicht weiter relevant, wesentlich ist lediglich, daß dazu gepulste Behandlungs-Laserstrahlung 2 verwendet wird. Beispielsweise kann ein Behandlungsgerät 1 verwendet werden, wie sie in der WO 2004/032810 A2 beschrieben ist. Wesentlich ist weiter, daß eine Vielzahl von Laserpulsfoki im Gewebe eine Schnittfläche ausbildet, deren Form vom Muster abhängt, mit dem die Laserpulsfoki im Gewebe angeordnet sind/werden. Das Muster gibt Zielpunkte für die Fokuslage vor, an denen ein oder mehrere Laserpuls(e) abgegeben wird(werden), und definiert die Form und Lage der Schnittfläche. Für die nachfolgend erläuterten Verfahren und Vorrichtungen ist das Muster der Zielpunkte von Bedeutung und wird noch näher beschrieben werden.

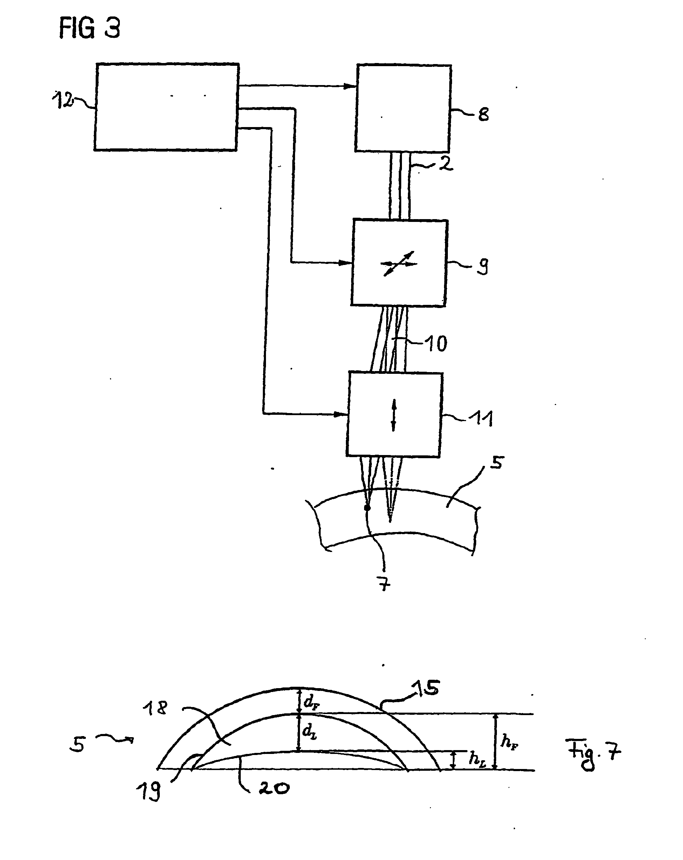

Um nun eine Fehlsichtigkeitskorrektur auszuführen, wird mittels der gepulsten Laserstrahlung aus einem Gebiet innerhalb der Hornhaut 5 Material entfernt, indem dort Gewebeschichten getrennt werden, die das Material isolieren und dann eine Materialentnahme ermöglichen. Die Materialentfernung bewirkt eine Volumenänderung in der Hornhaut, welche eine Änderung der optischen Abbildungswirkung der Hornhaut 5 zur Folge hat, die genau so bemessen ist, daß damit die zuvor ermittelte Fehlsichtigkeit möglichst korrigiert ist/wird. Zur Isolierung des zu entfernenden Volumens wird der Fokus der Laserstrahlung 2 auf Zielpunkte in der Hornhaut 5 gerichtet, in der Regel in einem Bereich, der unterhalb des Epithels und der Bowman'schen Membran sowie oberhalb der Decemetschen Membran und des Endothels liegt. Das Behandlungsgerät 1 weist dazu einen Mechanismus zum Verstellen der Lage des Fokus der Laserstrahlung 2 in der Hornhaut 5 auf. Dies ist schematisch in Figur 3 gezeigt.

In Figur 3 sind Elemente des Behandlungsgeräts 1 nur insoweit eingetragen, als sie zum Verständnis der Fokusverstellung erforderlich sind. Die Laserstrahlung 2 wird, wie bereits erwähnt, in einem Fokus 7 in der Hornhaut 5 gebündelt, und die Lage des Fokus 7 in der Hornhaut wird verstellt, so daß zur Schnittflächenerzeugung an verschiedenen Stellen fokussiert Energie aus Laserstrahlungspulsen in das Gewebe der Hornhaut 5 eingetragen wird. Die Laserstrahlung

2 wird von einem Laser 8 als gepulste Strahlung bereitgestellt. Ein xy-Scanner 9, der in einer Variante durch zwei im wesentlichen orthogonal ablenkende Galvanometerspiegel realisiert ist, lenkt den vom Laser 8 kommenden Laserstrahl zweidimensional ab, so daß nach dem xy-Scanner 9 ein abgelenkter Laserstrahl 10 vorliegt. Der xy-Scanner 9 bewirkt somit eine Verstellung der Lage des Fokus 7 im wesentlichen senkrecht zur Haupteinfallsrichtung der Laserstrahlung 2 in die Hornhaut 5. Zur Verstellung der Tiefenlage ist neben dem xy-Scanner 9 ein z-Scanner 11 vorgesehen, der beispielsweise als verstellbares Teleskop ausgebildet ist. Der z-Scanner 11 sorgt dafür, daß die z-Position der Lage des Fokus 7, d.h. dessen Position auf der optischen Achse des Einfalls verändert wird. Der z-Scanner 11 kann dem xy-Scanner 9 nach- oder vorgeordnet sein. Die nachfolgend mit x, y, z bezeichneten Koordinaten beziehen sich also auf die Ablenkung der Lage des Fokus 7.

Für das Funktionsprinzip des Behandlungsgerätes 1 ist die Zuordnung der einzelnen Koordinaten zu den Raumrichtungen nicht wesentlich, der einfacheren Beschreibung halber ist jedoch nachfolgend mit z immer die Koordinate entlang der optischen Achse des Einfalls der Laserstrahlung 2 bezeichnet, und x sowie y bezeichnen zwei zueinander orthogonale Koordinaten in einer Ebene senkrecht zur Einfallsrichtung des Laserstrahls. Dem Fachmann ist natürlich bekannt, daß eine dreidimensionale Beschreibung der Lage des Fokus 7 in der Hornhaut 5 auch durch andere Koordinatensysteme erfolgen kann, insbesondere muß es sich nicht um ein rechtwinkliges Koordinatensystem handeln. Daß der xy-Scanner 9 um zueinander rechtwinklige Achsen ablenkt ist also nicht zwingend, vielmehr kann jeder Scanner verwendet werden, der in der Lage ist, den Fokus 7 in einer Ebene zu verstellen, in der die Einfallsachse der optischen Strahlung nicht liegt. Somit sind auch schiefwinklige Koordinatensysteme möglich.

Weiter können auch nicht-geradlinige Koordinatensysteme zur Beschreibung bzw. Steuerung der Lage des Fokus 7 verwendet werden, wie dies nachfolgend auch noch erläutert wird. Beispiele für solche Koordinatensysteme sind Kugelkoordinaten (auch als sphärische Koordinaten bezeichnet) sowie zylindrische Koordinaten.

Zur Steuerung der Lage des Fokus 7 werden der xy-Scanner 9 sowie der z-Scanner 11, die gemeinsam ein konkretes Beispiel einer dreidimensionalen Fokusverstelleinrichtung realisieren, von einem Steuergerät 12 über nicht näher bezeichnete Leitungen angesteuert. Gleiches gilt für den Laser 8. Das Steuergerät 3 sorgt für einen geeignet synchronen Betrieb des Lasers 8 sowie der dreidimensionalen Fokusverstelleinrichtung, exemplarisch realisiert durch den xy-Scanner 9 sowie den z-Scanner 11, so daß die Lage des Fokus 7 in der Hornhaut 5 so verstellt wird, daß letztendlich ein Material bestimmten Volumens isoliert wird, wobei die spätere Volumenentfernung eine gewünschte Fehlsichtigkeitskorrektur bewirkt.

Das Steuergerät 12 arbeitet nach vorgegebenen Steuerdaten, welche die Zielpunkte für die Fokusverstellung vorgeben. Die Steuerdaten sind in der Regel in einem Steuerdatensatz zusammengefaßt. Dieser gibt in einer Ausführungsform die Koordinaten der Zielpunkte als Muster vor, wobei die Reihenfolge der Zielpunkte im Steuerdatensatz die Aneinanderreihung der Fokuslagen und damit letztlich eine Bahnkurve (hier auch verkürzt als Bahn bezeichnet) festlegt. Der Steuerdatensatz enthält in einer Ausführungsform die Zielpunkte als konkrete Stellwerte für den Fokuslagenverstellmechanismus, z.B. für den xy-Scanner 9 und den z-Scanner 11. Zur Vorbereitung des augenchirurgischen Verfahrens, also bevor das eigentliche Operationsverfahren ausgeführt werden kann, werden die Zielpunkte und vorzugsweise auch deren Reihenfolge im Muster bestimmt. Es muß eine Vorplanung des operativen Eingriffes dahingehend erfolgen, daß die Steuerdaten für das Behandlungsgerät 1 ermittelt werden, deren Anwendung dann eine für den Patienten 4 optimale Fehlsichtigkeitskorrektur erreicht.

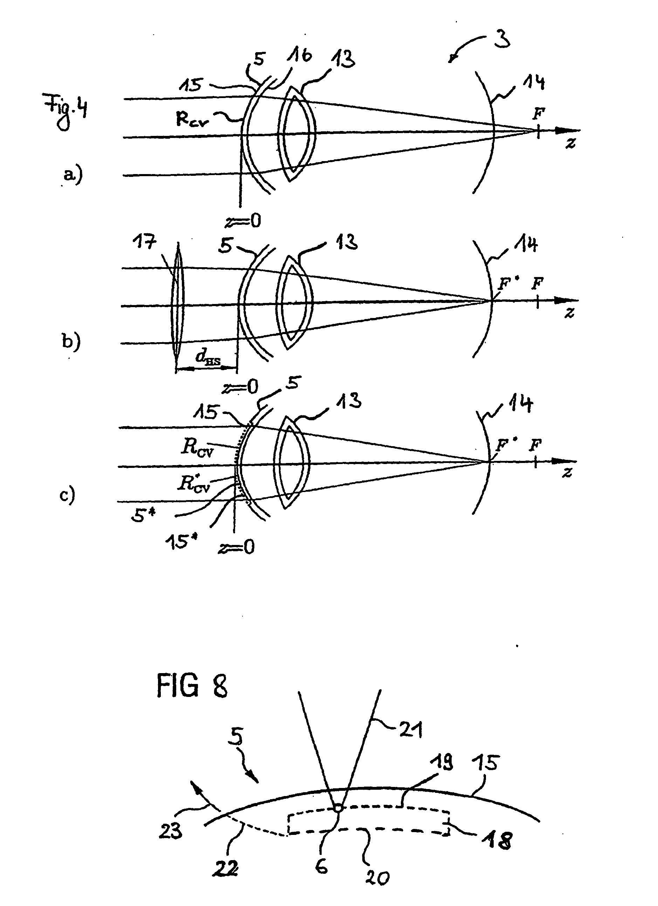

Zuerst gilt es das aus in der der Hornhaut 5 zu isolierende und später zu entfernende Volumen festzulegen. Wie bereits anhand Fig. 1a geschildert bedarf es dazu einer Feststellung des Korrekturbedarfs. Figur 4 zeigt in Teilfiguren a), b) und c) die optischen Verhältnisse am Auge 3 des Patienten 4. Ohne Fehlsichtigkeitskorrektur liegt die in Teilfigur a) gezeigte Situation vor. Die Hornhaut 5 bewirkt zusammen mit der Augenlinse 13 eine Fokussierung eines im Unendlichen liegenden Gegenstandes in einen Fokus F, der auf der z-Achse hinter der Netzhaut 14 liegt. Die abbildende Wirkung rührt dabei zum einen von der bei nichtakkomodiertem Auge entspannten Augenlinse 13 sowie zum anderen von der Augenhornhaut 5 her, die im wesentlichen durch eine Hornhautvorderfläche 15 sowie eine Hornhautrückseite 16 definiert ist und aufgrund ihrer Krümmung ebenfalls eine abbildende Wirkung hat. Die optische Wirkung der Hornhaut 5 ist durch den Krümmungsradius $R_{CV}$ der Hornhautvorderfläche bedingt. Teilfigur a) stellt die Fehlsichtigkeit nur exemplarisch dar, real können die oben erwähnten komplexeren Fehlsichtigkeiten vorliegen. Für sie gilt die nachfolgenden Beschreibung jedoch ebenfalls, allerdings können die angegebenen Gleichungen dann mitunter eine zusätzliche Winkelabhängigkeit beinhalten, auch wenn darauf nicht ausdrücklich hingewiesen wird.

Zur Fehlsichtigkeitskorrektur wird bekannter Weise, wie in Teilfigur b) der Figur 4 dargestellt, eine Vorsatz-Linse 17 in Form einer Brille im Abstand $d_{HS}$ vom Scheitelpunkt der Hornhaut 5 vor das Auge 3 gesetzt. Die Linse 17 der Brille ist in ihrer Brechkraft $B_{BR}$ so angepaßt, daß sie den Fernpunkt des gesamten Systems, d.h. aus Brille und Auge, vom Fokuspunkt F zum korrigierten Fokuspunkt F* verschiebt, der auf der Netzhaut 14 liegt.

Hinsichtlich der in dieser Beschreibung verwendeten Nomenklatur sei angemerkt, daß durch die Anfügung eines Sterns an Größen verdeutlicht wird, daß es sich um Größen handelt, die nach einer Korrektur erhalten werden. Der Fokus F* ist also derjenige Fokus, der nach der optischen Korrektur vorliegt, die in der Teilfigur b) der Figur 4 durch die Linse 17 der Brille erreicht wird.

Unter der gerechtfertigten Annahme, daß eine Dickenänderung der Hornhaut 5 im wesentlichen den Krümmungsradius der Luft zugewandten Hornhaut-Vorderseite 15 modifiziert, nicht aber den Krümmungsradius der dem Augeninneren zuliegenden Hornhautrückseite 16, wird durch die Volumenentfernung der Krümmungsradius $R_{CV}$ der Hornhautvorderseite 15 modifiziert. Die um das Volumen verminderte Hornhaut 5 hat eine derart geänderte Abbildungswirkung, daß der dann korrigierte Fokus F* auf der Netzhaut 14 liegt. Nach der Korrektur liegt eine veränderte Hornhautvorderfläche 15* vor, und es ist eine Fehlsichtigkeitskorrektur auch ohne Brille erreicht.

Zur Bestimmung des Musters der Zielpunkte wird deshalb die zu erreichende Krümmung der modifizierten Hornhautvorderfläche 15* ermittelt. Dabei ist Ausgangspunkt die Brechkraft der Linse 17 der Brille, da die Ermittlung der entsprechenden Parameter ein Standardverfahren in der Augenoptik ist. Für die Brechkraft $B_{BR}(\varphi)$ der Linse 17 der Brille gilt folgende Formel:

$$(1) \qquad B_{BR}(\varphi) = Sph + Cyl \cdot \sin^2(\varphi - \theta).$$

In dieser Gleichung bezeichnen Sph und Cyl die zu realisierenden Korrekturwerte spärischen bzw. astigmatischen Brechungsfehler und $\theta$ die Lage der Zylinderachse der zylindrischen (astigmatischen) Fehlsichtigkeit, wie sie dem Fachmann in der Optometrie bekannt sind. Der Parameter $\varphi$ schließlich bezieht sich auf ein Zylinderkoordinatensystem des Auges und wird auf das Auge schauend entgegen dem Uhrzeigersinn gezählt, wie es in der Augenoptik üblich ist. Mit dem Wert $B_{BR}$ wird nun die Krümmung der modifizierten Hornhautvorderfläche 15* wie folgt eingestellt:

$$(2) \qquad R_{CV}^* = 1 / ( (1/R_{CV}) + B_{BR} / ( (n_c-1) \cdot (1 - d_{HS} \cdot B_{BR}))) + F$$

In Gleichung (2) bezeichnet $n_c$ die Brechkraft des Materials der Hornhaut. Der entsprechende Wert liegt üblicherweise bei 1,376; $d_{HS}$ bezeichnet den Abstand, in dem eine Brille mit der Brechkraft $B_{BR}$ vom Hornhautscheitel liegen muß, um die gewünschte Fehlsichtigkeitskorrektur mittels Brille zu erzeugen; $B_{BR}$ bezeichnet die zuvor erwähnte Brechkraft der Brille gemäß Gleichung (1). Die Angabe für die Brechkraft $B_{BR}$ kann auch Fehlsichtigkeiten erfassen, die über eine normale sphärische oder zylindrische Korrektur hinausgehen. $B_{BR}$ (und damit automatisch auch $R_{CV}^*$) haben dann zusätzliche Koordinatenabhängigkeiten.

Der Korrektur-Faktor F berücksichtigt die optische Wirkung der Dickenänderung der Hornhaut aus und kann in erster Näherung als konstanter Faktor angesehen werden. Für eine hochgenaue Korrektur kann der Faktor gemäß folgender Gleichung errechnet werden:

$$(3) \qquad F = (1 - 1/n_c) \cdot (d_C^* - d_C).$$

$d_C$ bzw. $d_C^*$ ist dabei die Hornhautdicke vor bzw. nach der optischen Korrektur. Für eine genaue Bestimmung erfolgt eine Berechnung von $R_{CV}^*$ iterativ, indem bei der i-ten Berechnung aus der Differenz $(R_{CV}^* - R_{CV})$ auf die Größe $(d_C^* - d_C)$ geschlossen wird und das entsprechende daraus erhaltene Ergebnis für die Dickenänderung bei der (i+1)-ten Berechnung angewendet wird. Dies kann man so lange durchführen, bis ein Abbruchkriterium erfüllt wird, beispielsweise wenn die Differenz des Ergebnisses für die Dickenänderung bei zwei aufeinanderfolgenden Iterationsschritten unter einer entsprechend festgelegten Grenze liegt. Diese Grenze kann beispielsweise über eine konstante Differenz festgelegt werden, die einer für die Behandlung angemessene Genauigkeit der Refraktionskorrektur entspricht.

Vernachlässigt man die Dickenänderung der Augenhornhaut, was für ein vereinfachtes Verfahren durchaus zulässig ist, kann der Korrektur-Faktor F in Gleichung (2) für eine vereinfachte Berechnung auch gleich Null gesetzt, also vernachlässigt und weggelassen werden. Man erhält überraschenderweise folgende einfache Gleichung für die Brechkraft der modifizierten Hornhaut 5*:

$$B_{CV}^* = B_{CV} + B_{BR} / (1 - B_{BR} \cdot d_{HS})$$

Aus dieser Gleichung ergibt sich für den Fachmann auf einfache Art und Weise mittels der Gleichung $B_{CV}^* = (n-1) / R_{CV}^*$ der Radius $R_{CV}^*$ der Hornhautvorderfläche 15*, der nach der Modifikation vorliegen muß, um die gewünschte Fehlsichtigkeitskorrektur zu erhalten, zu: $R_{CV}^* = 1 / ((1/R_{CV}) + B_{BR} / ((n_c-1) (1 - d_{HS} \cdot B_{BR})))$.

Für das Volumen, dessen Entfernung die obige Krümmungsänderung der Hornhautvorderfläche 15 bewirkt, wird nun die das Volumen isolierende Grenzfläche festgelegt. Dabei ist vorzugsweise zu berücksichtigen, daß sich der Durchmesser des zu korrigierenden Bereichs und damit der Durchmesser des zu entnehmenden Volumens möglichst über die Pupillengröße bei dunkelangepaßtem Auge erstrecken sollte.

In einer ersten Variante wird mittels dem Fachmann bekannter numerischer Methoden eine Freifläche definiert werden, die ein Volumen umschreibt, dessen Entfernung die Krümmungsänderung bewirkt. Dazu wird entlang der z-Achse die Dickenänderung ermittelt, die zur gewünschten Krümmungsmodifikation nötig ist. Daraus ergibt sich das Volumen als Funktion von r, $\varphi$ (in Zylinderkoordinaten) und daraus wiederum dessen Grenzfläche.

Eine einfache analytische Rechnung liefert die folgende zweite Variante, bei der die Grenzfläche des Volumens durch zwei Teilflächen aufgebaut wird, eine zur Hornhautoberfläche 15 hinliegende anteriore Teilfläche und eine gegenüberliegende posteriore Teilfläche. Die entsprechenden Verhältnisse zeigt Figur 5. Das Volumen 18 ist zur Hornhautvorderfläche 15 hin durch eine anteriore Schnittfläche 19 begrenzt, die in konstantem Abstand $d_F$ unter der Hornhautvorderfläche 15 liegt. Diese anteriore Schnittfläche 19 wird in Analogie zur Laserkeratomen auch als Flap-Fläche 19 bezeichnet, da sie dort dazu dient, in Kombination mit einem Öffnungsschnitt zum Rand hin die Augenhornhaut 5 eine Lamelle in Form eines "Flap" von der darunterliegenden Hornhaut 5 abheben zu können. Diese Art der Entnahme des zuvor isolierten Volumens 18 ist natürlich auch hier möglich.

Die anteriore Schnittfläche 19 hat einen Krümmungsverlauf, der um $d_F$ unter der Hornhautvorderfläche 15 liegt. Ist diese sphärisch, kann für die Flap-Fläche 19 ein Krümmungsradius angegeben werden, der um $d_F$ geringer ist als der Krümmungsradius $R_{CV}$. Wie später für bevorzugte Varianten beschrieben wird, kann bei der Erzeugung der Schnittfläche 19 durch ein Kontaktglas dafür gesorgt werden, daß die Hornhautvorderfläche 15 zum Zeitpunkt der Schnittflächenerzeugung sphärisch ist, so daß das Muster der Zielpunkte eine sphärische Schnittfläche bewirkt. Die Relaxation des Auges 3 nach Abnahme des Kontaktglases mag dann zwar zu einer nicht-sphärischen Schnittfläche 19 führen, sie hat aber dennoch konstanten Abstand zur Hornhautvorderfläche 15 bzw. 15*.

Posterior ist das Volumen 18, das aus der Hornhaut 5 entfernt werden soll, durch eine posteriore Schnittfläche 20 begrenzt, die im Allgemeinen keinen konstanten Abstand zur Hornhautvorderfläche 15 hat. Die posteriore Schnittfläche 20 wird deshalb so ausgebildet sein, daß das Volumen 18 in Form eines Lentikels vorliegt, weshalb die posteriore Schnittfläche 20 auch als Lentikel-Fläche 20 bezeichnet wird. In Figur 5 ist sie exemplarisch für eine Myopiekorrektur als ebenfalls sphärische Fläche mit einem Krümmungsradius $R_L$ eingezeichnet, wobei im Allgemeinen das Zentrum dieser Krümmung nicht mit dem Krümmungszentrum der in Figur 5 ebenfalls sphärischen Hornhautvorderfläche 15 zusammenfällt. Bei einer Hyperopiekorrektur ist $R_L$ größer als $R_{CF} - d_F$.

Figur 6 zeigt die Verhältnisse nach Entfernung des Volumens 18. Der Radius der modifizierten Hornhautvorderfläche 15* beträgt nun $R_{CV}*$ und kann beispielsweise gemäß den zuvor beschriebenen Gleichungen berechnet werden. Die zentrale Dicke $d_L$ des entnommenen Volumens 18 ist dabei maßgeblich für die Radiusänderung, wie Figur 7 verdeutlicht. In dieser Figur sind als weitere Größen noch die Höhe $h_F$ der durch die anteriore Schnittfläche 19 definierten Kugelkappe, die Höhe $h_L$ der durch die posteriore Schnittfläche 20 definierten Kugelkappe sowie die Dicke $d_L$ des zu entfernenden Volumens 18 eingezeichnet.

Die posteriore Schnittfläche 20 legt aufgrund des konstanten Abstandes zwischen Hornhautvorderfläche 15 und anteriorer Schnittfläche 19 den Krümmungsverlauf der Hornhautvorderfläche 15* nach Entfernung des Volumens 18 fest. Somit wird die posteriore Schnittfläche 20 z.B. bei einer zylindrische Parameter berücksichtigenden Fehlsichtigkeitskorrektur einen winkelabhängigen Krümmungsradius haben. Für die in Figur 7 gezeigte Lentikel-Fläche 20 gilt allgemein:

$$R_L(\varphi) = R_{CV}^{\cdot}{}^*(\varphi) - d_F \, ,$$

bzw. in Zylinderkoordinaten (z, r, $\varphi$)

$$z_L(r, \varphi) = R_L(\varphi) - (R_L{}^2(\varphi) - r^2)^{1/2} + d_L + d_F.$$

Ohne Berücksichtigung eines Astigmatismus entfällt die Abhängigkeit von $\varphi$ und die Lentikel-Fläche 20 ist sphärisch. Die Lentikel-Fläche 20 besitzt aber, geht man vom Bedarf für eine Zylindrische Fehlsichtigkeitskorrektur aus, in der Regel auf verschiedenen Achsen unterschiedliche Krümmungsradien, wobei diese natürlich meist den gleichen Scheitelpunkt haben.

Damit wird weiter automatisch deutlich, daß im Fall einer myopischen Zylinderkorrektur die theoretische Schnittlinie zwischen Flap-Fläche 19 und Lentikel-Fläche 20 nicht in einer Ebene, d.h. bei konstanten z-Koordinaten liegt. Der kleinste Krümmungsradius der Lentikel-Fläche 20 liegt bei $\varphi = \theta + \pi/2$, der größte natürlich auf der Achse $\theta$ der zylindrischen Fehlsichtigkeit, d.h. bei $\varphi = \theta$. Bei einer Übersichtigkeitskorrektur fallen anders bei der Darstellung der Figur 7 der Scheitelpunkt von Flap-Fläche 19 und Lentikel-Fläche 20 theoretisch zusammen und die Lentikel-Fläche 20 ist stärker gekrümmt, als die Flap-Fläche 19. Die Dicke $d_L$ des Lentikels ergibt sich als zentrale Lentikeldicke bei Myopie.

Das als Lentikel aufzufassende Volumen 18 hat im Falle der Myopiekorrektur am Rand theoretisch eine Schnittlinie von Lentikel-Fläche 20 und Flap-Fläche 19. Bei Hyperopiekorrektur ist immer eine endliche Randdicke gegeben, da die Lentikel-Fläche 20 schwächer gekrümmt ist, als die Flap-Fläche 19. Hier aber ist theoretisch die zentrale Lentikeldichte gleich Null.

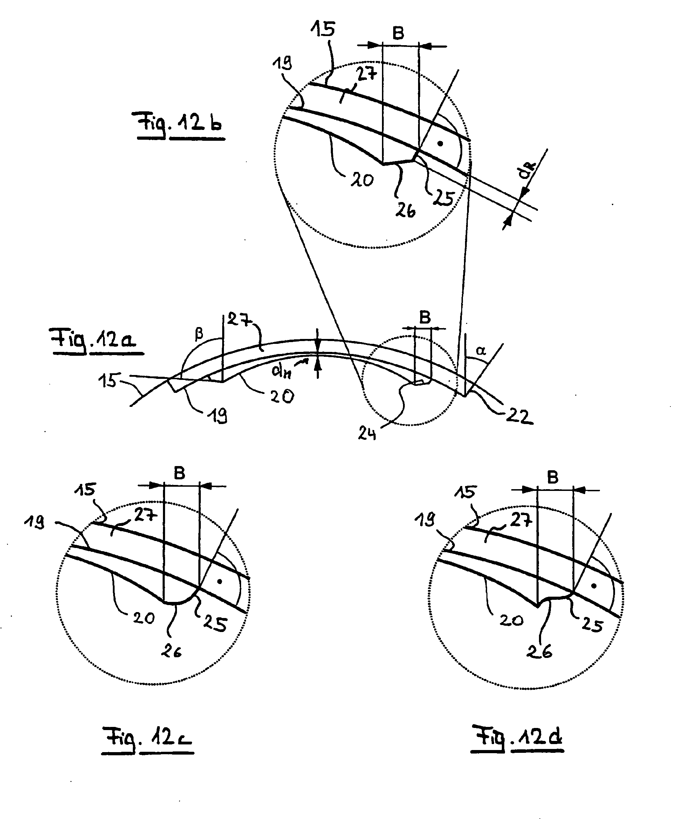

Neben der Flap-Fläche 20 und der Lentikel-Fläche 19 ist eine zusätzliche Randfläche vorgesehen, welche das begrenzte Volumen 18 von Flap-Fläche 20 und der Lentikel-Fläche 19 am Rand begrenzt. Der Schnitt dieser Randfläche wird ebenfalls mit dem gepulsten Laserstrahl ausgeführt. Die Struktur der Randfläche wird nachfolgend anhand der Figuren 12a-c erläutert.

Die in den Figuren gezeigte Ausbildung des Volumens 18 als durch eine anteriore Schnittfläche 19 mit konstantem Abstand zur Hornhautvorderfläche 15 sowie eine posteriore Schnittfläche 20 begrenzt, ist nur eine Variante zur Begrenzung des Volumens 18. Sie hat jedoch den Vorteil, daß die optische Korrektur wesentlich nur durch eine Fläche (die Lentikelfläche 20) festgelegt wird, so daß die analytische Beschreibung der anderen Teilfläche der Grenzfläche einfach ist.

Weiter sind optimale Sicherheitsmargen hinsichtlich des Abstandes des Volumens zur Hornhautvorderfläche 15 und Hornhautrückfläche 16 gegeben. Die Restdicke $d_F$ zwischen anteriorer Schnittfläche 19 und Hornhautvorderfläche 15 kann konstant auf einen Wert von beispielsweise 50 bis 200 $\mu$m eingestellt werden. Insbesondere kann sie so gewählt sein, daß das schmerzempfindliche Epithel in der Lamelle verbleibt, die durch die Flap-Fläche 19 unter der Hornhautvorderfläche 15 gebildet ist. Auch steht die Ausbildung der sphärischen Flap-Fläche 19 in Kontinuität mit bisherigen Keratometerschnitten, was für die Akzeptanz der Methode vorteilhaft ist.

Nach Erzeugen der Schnittflächen 19 und 20 wird dann das derart isolierte Volumen 18 aus der Hornhaut 5 entfernt. Dies ist schematisch in Figur 8 dargestellt, die zudem verdeutlicht, daß die Schnittflächen 19 und 20 durch Einwirkung des in einem Fokuskegel 21 einfallenden Behandlungslaserstrahls erzeugt werden, beispielsweise durch Aneinanderreihung von Plasmablasen, so daß in einer bevorzugten Ausführungsform die Flap-Schnittfläche 19 und die Lentikel-Schnittfläche 20 durch geeignete dreidimensionale Verstellung der Fokuslage der gepulsten Laserstrahlung 2 erzeugt werden.

Alternativ kann in einer vereinfachten Ausführungsform aber auch lediglich die Flap-Fläche 19 durch Zielpunkte, die die gekrümmte Schnittfläche 19 in konstantem Abstand zu Hornhautvorderfläche 15 definieren mittels gepulster Laserstrahlung gebildet werden und die Entfernung des Volumens 18 erfolgt durch Laserablation, beispielsweise durch Verwendung eines Excimers-Laserstrahls. Hierzu kann die Lentikel-Fläche 20 als Grenzfläche des Abtrages definiert werden, auch wenn das nicht zwingend erforderlich ist. Das Behandlungsgerät 1 arbeitet dann wie ein bekanntes Laserkeratom, allerdings wird die Schnittfläche 19 an gekrümmter Hornhaut erzeugt. Die vorangehend bzw. nachfolgend beschriebenen Merkmale sind auch in solchen Varianten möglich, insbesondere was die Bestimmung der Begrenzungsfläche, deren geometrische Definition und die Ermittlung von Steuerparametern angeht.

Erzeugt man sowohl die Lentikel-Fläche 20 als auch die Flap-Fläche 19 mittels gepulster Laserstrahlung, ist es zweckmäßig, die Lentikel-Fläche 20 vor der Flap-Fläche 19 auszubilden, da das optische Ergebnis bei der Lentikel-Fläche 20 besser (wenn nicht überhaupt erst zu erreichen) ist, wenn oberhalb der Lentikel-Fläche 20 noch keine Veränderung der Hornhaut 5 eintrat.

Das Entfernen des durch die gepulste Laserstrahlung isolierten Volumens 18 kann, wie in Figur 8 angedeutet, durch einen Randschnitt 22 erreicht werden, der es erlaubt, das Volumen 18 in Richtung eines in Figur 8 eingezeichneten Pfeils 23 herauszuziehen. Alternativ kann der Randschnitt 22 aber so ausgebildet werden, daß er die anteriore Schnittfläche 19, d. h. die Flap-Fläche 19, in Form eines Ringes mit der Hornhautvorderfläche 15 verbindet, wobei der Randschnitt allerdings nicht vollständig um einen Winkel von 360° umläuft. Die derart isolierte Lamelle bleibt in einem schmale Bereich mit dem übrigen Gewebe der Hornhaut 5 in Verbindung. Diese Verbindungsbrücke dient dann als Gelenk, um die ansonsten isolierte Lamelle von der Hornhaut 5 abzuklappen und das dadurch zugängige, bereits isolierte Volumen 18 vom Rest der Augenhornhaut 5 abnehmen zu können. Die Lage der Verbindungsbrücke ist bei Erzeugung der Steuerdaten bzw. der Zielpunkte vorgebbar. Das beschriebene Vorgehen bzw. Gerät realisiert also unter diesem Gesichtspunkt die Isolierung des Volumens 19 innerhalb der Hornhaut 5 und das Erzeugen einer mit der restlichen Augenhornhaut über eine Gewebebrücke verbundenen Lamelle als Deckel über dem Volumen. Der Deckel kann abgeklappt und das Volumen 18 entnommen werden.

Für die Erzeugung der Schnittflächen 19 und 20 können die Zielpunkte nun auf verschiedenste Art und Weise angeordnet werden. Im Stand der Technik ist beispielsweise in der WO 2005/011546 zur Erzeugung von Schnittflächen in der Augenhornhaut beschrieben, daß spezielle Spiralen eingesetzt werden können, die beispielsweise um eine im wesentlichen senkrecht zur optischen Achse (z-Achse) liegende Hauptachse in Art einer Schraubenlinie verlaufen. Auch ist die Verwendung eines Scanmusters bekannt, das die Zielpunkte zeitenweise anordnet (vgl. WO 2005/011545). Diese Möglichkeiten können selbstverständlich zur Erzeugung der oben definierten Schnittflächen verwendet werden.

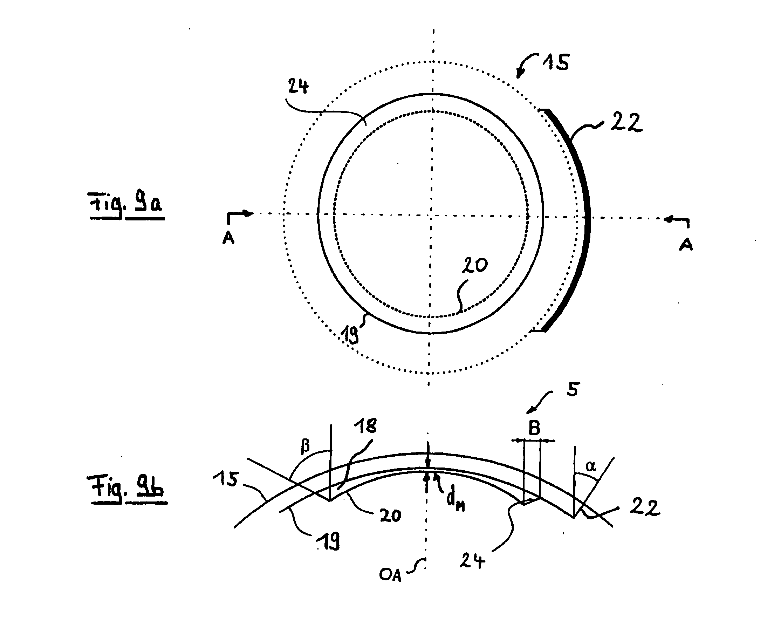

Die oben bereits erwähnte Randfläche ist in den Figuren 9a und 9b genauer zu sehen. Elemente, die bereits anhand anderer Figuren erläutert wurden, sind in diesen Figuren mit denselben Bezugszeichen versehen, so daß auf ihre Erläuterung gegebenenfalls verzichtet wird.

Figur 9a zeigt eine Draufsicht auf die Hornhautvorderfläche 15 mit der Flap-Fläche 19 sowie der Lentikel-Fläche 20 (deren Kante gestrichelt gezeichnet ist) sowie dem Randschnitt 22 im Falle einer Hyperopiekorrektur. Weiter ist in Figur 9a eine Übergangszone zwischen dem Rand der Lentikel-Fläche 20 und der Flap-Fläche 19 zu erkennen, die durch eine Randfläche 24 erreicht ist. Diese Randfläche 24 ist in Figur 9b deutlich zu sehen, die eine Schnittdarstellung durch die Darstellung der Figur 9a entlang der Linien A-A ist.

Die Randfläche 24 bewirkt den Übergang von der Lentikel-Fläche 20 zur Flap-Fläche 19. Sie ist dabei in der Ausführungsform der Figur 9b als konische Fläche ausgeführt, die schräger liegt, als eine (in Draufsicht auf Fig. 9a) konische Fläche, welche senkrecht zur Hornhautvorderfläche 15 oder zur parallel dazu verlaufenden Flap-Fläche 19 läge. Einen solchen Verlauf hat beispielsweise der Randschnitt 22, der bezogen auf die Seh-Achse OA in einem Winkel $\alpha$ liegt, der den erwähnten senkrechten Verlauf zur Hornhautvorderfläche zur Folge hat.

Die Randfläche 24 verläuft hingegen geneigter, so daß die Breite B, welche die Randfläche 24 bei Draufsicht in Richtung der optischen Achse OA hat, größer ist, als z. B. beim Randschnitt 24. Der entsprechende Winkel $\beta$ ist folglich ebenfalls größer als der Winkel $\alpha$.

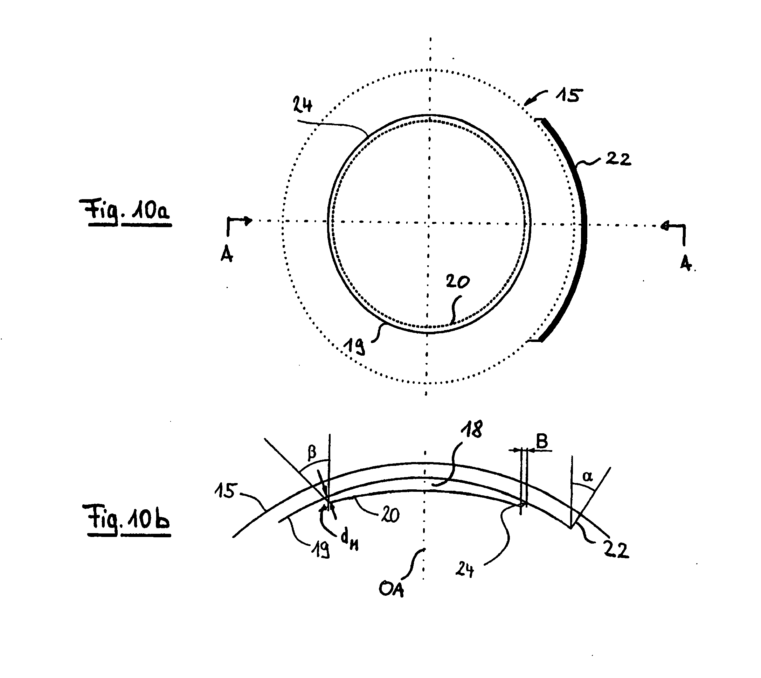

Die Figuren 10a und 10b zeigen die entsprechenden Verhältnisse im Falle eines myopen Lentikels, d.h. also bei der Myopie-Korrektur. Auch hier ist die Randfläche 24 vorhanden und führt zu einer endlichen Randdicke des Lentikels 18, welche an und für sich aufgrund der größeren Krümmung der Lentikel-Fläche 20 gegenüber der Flap-Fläche 10 nicht gegeben wäre, da beide Flächen zumindest in ihrer Fortsetzung eine Schnittlinie haben würden, also der Rand in einer Schnittlinie endete.

Die Mindestdicke des Lentikels 18, welche bei einer hyperopen Form gem. Figur 9b an oder nahe der optischen Achse OA vorliegt, besteht bei einem myopen Lentikel gem. Figur 10b also am Rand. Entsprechend ist die Mindestdicke $d_M$ in Fig. 10b auch am Rand eingezeichnet.

Selbstverständlich können die Schnittdarstellungen der Figuren 9b und 10b nur dann beschreibend für das gesamte Lentikel sein, wenn keine Korrektur höherer Ordnung, insbesondere kein Astigmatismus, vorliegt. Ist solches gegeben, ist die Lentikelfläche 20 von Sphärizität abweichend entsprechend korrigiert, was sich auf für den Fachmann naheliegende Weise auch auf die Randfläche 24 auswirkt.

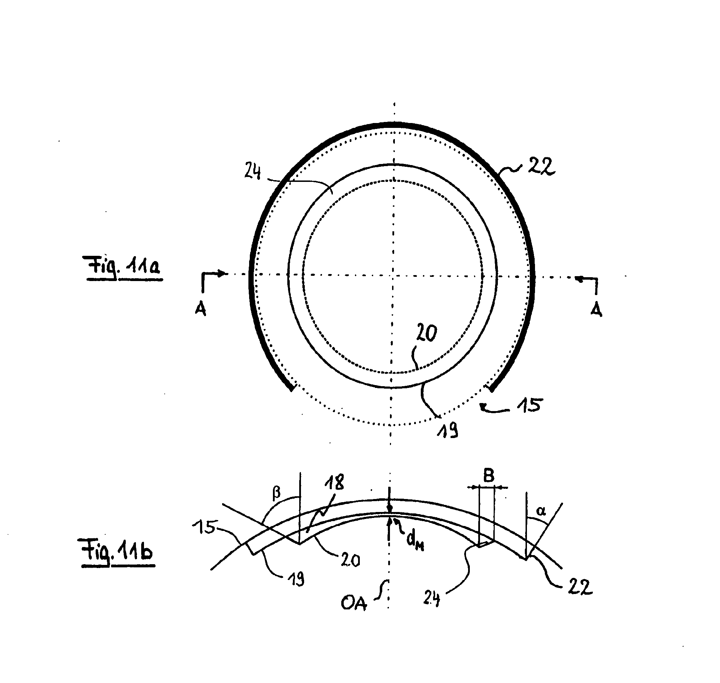

Die Figuren 11 a und 11 b zeigen schließlich Verhältnisse entsprechend denen deren Figuren 9a und 9b, jedoch verläuft hier der Randschnitt 22, welcher zur Freilegung des Lentikels 18 erzeugt wird, über einen sehr viel größeren Winkelbereich, als in Figuren 9 und 10.

Die Figuren 9 bis 11 zeigen eine Randfläche 24, die als schräger Schnitt ausgeführt ist. Die Randfläche 24 kann jedoch auch eine von einem solchen, in Querschnittansicht geradlinigen Verlauf abweichende Struktur haben. Dies ist exemplarisch in den Figuren 12a bis d gezeigt. Hierin gibt Figur 12a eine Schnittdarstellung ähnlich der der Figuren 9b, 10b oder 11b wieder. Die Figuren 12b bis 12d zeigen den in der Figur 12a punktierten Ausschnitt vergrößert und stellen unterschiedliche Varianten für die Struktur der Randfläche 24 dar.

Gemäß Figur 12b besteht die Randfläche 24 aus zwei im Querschnitt im wesentlichen geradlinigen Abschnitten 25 und 26. Der erste Abschnitt 25 läuft senkrecht in die Flap-Fläche 19 ein. Die Höhe des ersten Abschnittes 25 bewirkt eine Dicke $d_R$ des Randes. Diese Dicke ist vorzugsweise im Bereich von 5 bis 10 $\mu$m gewählt und garantiert, daß beim Entnehmen des Lentikels 18 kein Abreißen von Bruchstücken im Bereich der Randfläche 24 auftritt. Solche Bruchstücke hätten erheblich nachteilige Auswirkungen auf das Einwachsen und würden zu einer unerwünschten Regression führen. Um trotz des senkrecht zur Flap-Fläche 19 liegenden ersten Abschnittes 25 ein sanftes Anlegen der durch den Flap-Schnitt 19 isolierten Hornhautlamelle 27 zu erreichen, wenn das lentikelförmige Volumen entnommen wurde, hat der zweite Abschnitt 26 der Randfläche 24 in der Ausführungsform der Figur 12b einen schrägen Verlauf zur Seh-Achse hin. Durch diesen schrägen Verlauf ist wiederum eine Breite B erreicht, die deutlich größer als bei einem durchgängig senkrecht zur Flap-Fläche 19 verlaufenden Rand 24 gegeben wäre.

Figur 12c zeigt eine Abwandlung der Struktur der Randfläche 24 der Figur 12b, bei der sich an den senkrecht in die Flap-Fläche 19 einlaufenden ersten Abschnitt 25 ein kontinuierlich gekrümmter zweiter Abschnitt 26 ausgebildet ist, durch den der Rand zwischen der Lentikel-Fläche 20 und der Flap-Fläche 19 im Bereich, der von der Augenhornhautvorderfläche 15 abgewandt ist, abgerundet ist. Das senkrechte Einlaufen des ersten Abschnittes 25 stellt wiederum eine Mindestranddicke $d_R$ sicher, die ein Abreißen von Gewebestücken am Lentikelrand bei der Entnahme des Lentikelgewebes verhindert.

Dies ist auch bei der Randstruktur gem. Figur 12d erreicht, die S-förmig ausgebildet ist, wobei wiederum der erste Abschnitt 25 senkrecht in die Flap-Fläche 19 einläuft. Aufgrund der S-Struktur weist die Schnittdarstellung der Randfläche 24 in dieser Ausgestaltung einen Wendepunkt auf, und der zweite Abschnitt 26 endet vorzugsweise ebenfalls rechtwinklig in der Lentikel-Fläche 20.

Das Kontaktglas hat den weiteren Vorteil, daß durch das Anpressen an die sphärische Kontaktglasunterseite 26 automatisch auch die Hornhautvorderfläche 15 sphärisch ist. Die in konstantem Abstand unter der Hornhautvorderfläche 15 liegende anteriore Schnittfläche 19 ist damit bei angepreßtem Kontaktglas ebenfalls sphärisch, was zu erheblich vereinfachter Ansteuerung führt. Es ist deshalb völlig unabhängig von anderen Merkmalen bevorzugt, ein Kontaktglas mit sphärischer Kontaktglasunterseite zu verwenden und das Volumen durch eine anteriore Schnittfläche 19 sowie eine posteriore Schnittfläche zu begrenzen, wobei die anteriore Schnittfläche als sphärische Fläche in konstantem Abstand $d_F$ unter der Hornhautvorderfläche 15 erzeugt wird. Die posteriore Schnittfläche hat einen Krümmungsverlauf, welcher bei relaxiertem Auge, also nach Abnehmen des Kontaktglases, bis auf den Abstand $d_F$ zur Hornhautvorderfläche dem zur Fehlsichtigkeitskorrektur gewünschten entspricht. Analoges gilt für die Definition der Zielpunkte.

## Patentansprüche

1. Behandlungsvorrichtung zur operativen Hyperopiekorrektur am Auge (3), wobei die Behandlungsvorrichtung (1) eine von einer Steuereinrichtung (12) gesteuerte Lasereinrichtung (L) aufweist, welche durch Einstrahlen von Laserstrahlung (2) Hornhaut-Gewebe trennt, wobei die Steuereinrichtung (12) ausgebildet ist, die Lasereinrichtung (L) zur Abgabe der Laserstrahlung (2) in die Hornhaut (5) so anzusteuern, daß damit ein lentikelförmiges Volumen (18) in der Hornhaut (5) isoliert wird, dessen Entfernung aus der Hornhaut (18) die gewünschte Hyperopiekorrektur bewirkt, wobei die Steuereinrichtung (12) eingerichtet ist, bei der Ansteuerung der Lasereinrichtung (L) das lentikelförmige Volumen (18) so vorzugeben, daß es eine posteriore Fläche (20) und eine anteriore Fläche (19) hat, wobei letztere zumindest in einer Hauptebene (A-A) schwächer gekrümmt ist, als die posteriore Fläche (20), und deren Ränder über eine ringförmige Randfläche (24) verbunden sind, wobei eine Schnittkurve aus der Randfläche (24) und einer Ebene, in der die Sehachse (OA) enthalten ist, quer zur Sehachse (OA) eine Breite (B) hat, die größer als diejenige ist, welche in der gleichen Ebene eine gerade Strecke hätte, die am Rand der posterioren oder der anterioren Fläche (20, 19) auf der jeweiligen Fläche senkrecht steht und die anteriore Fläche (19) mit der posterioren Fläche (20) verbindet, **dadurch gekennzeichnet, daß** die Randfläche (24) einen ersten senkrecht in die anteriore Fläche (19) mündenden Abschnitt (25) und einen zweiten, stärker zur Sehachse (OA) hin geneigten Abschnitt (26) aufweist.

2. Verfahren zum Erzeugen von Steuerdaten für eine Lasereinrichtung (L) einer Behandlungsvorrichtung (1) zur operativen Hyperopiekorrektur am Auge (3), welche durch Einstrahlen von Laserstrahlung (2) Hornhaut-Gewebe trennt, wobei die Steuerdaten im Betrieb die Lasereinrichtung (L) zur Abgabe der Laserstrahlung (2) in die Hornhaut (5) so ansteuern, daß damit ein lentikelförmiges Volumen (18) in der Hornhaut (5) isoliert wird, dessen Entfernung aus der Hornhaut (18) die gewünschte Hyperopiekorrektur bewirkt, wobei die Steuerdaten das lentikelförmige Volumen (18) so vorgeben, daß es eine posteriore Fläche (20) und eine anteriore Fläche (19) hat, wobei letztere zumindest in einer Hauptebene (A-A) schwächer gekrümmt ist, als die posteriore Fläche (20), und deren Ränder über eine ringförmige Randfläche (24) verbunden sind, wobei eine Schnittkurve aus der Randfläche (24) und einer Ebene, in der die Sehachse (OA) enthalten ist, quer zur Sehachse (OA) eine Breite (B) hat, die größer als diejenige ist, welche in gleicher Ebene eine gerade Strecke hätte, die am Rand der posterioren oder der anterioren Fläche (20, 19) auf der jeweiligen Fläche senkrecht steht und die anteriore Fläche (19) mit der posterioren Fläche (20) verbindet, **dadurch gekennzeichnet, daß** die Randfläche (24) einen ersten senkrecht in die anteriore Fläche mündenden Abschnitt (25) und einen zweiten, stärker zur Sehachse (OA) hin geneigten Abschnitt (26) aufweist.

3. Vorrichtung oder Verfahren nach einem der obigen Ansprüche, **dadurch gekennzeichnet, daß** die anteriore Fläche (19) in einem konstantem Abstand $d_F$ zur Hornhautvorderfläche (15) liegt und die posteriore Fläche gekrümmt ist und einen Krümmungsradius $R_L = R_{CV}{}^* - d_F$ hat, wobei bei der Bestimmung der Steuerdaten folgende Gleichung verwendet wird:

$$R_{CV}{}^* = 1 \,/\, (\,(1/R_{CV}) + B_{BR} \,/\, (\,(n_c\text{-}1) \cdot (1 - d_{HS} \cdot B_{BR}))) + F,$$

und $R_{CV}$ der Krümmungsradius der Hornhaut (5) vor Entfernung des Volumens (18), $n_c$ die Brechkraft des Materials der Hornhaut (5), F ein Korrektur-Faktor ist, $B_{BR}$ die Brechkraft einer für die Fehlsichtigkeitskorrektur tauglichen Brille (17), sowie $d_{HS}$ der Abstand ist, in dem die Brille (17) mit der Brechkraft $B_{BR}$ vor dem Hornhautscheitel liegen müßte, um die gewünschte Fehlsichtigkeitskorrektur mittels der Brille (17) zu erreichen.

4. Vorrichtung oder Verfahren nach Anspruch 3, **dadurch gekennzeichnet, daß**

$$F = (1 - 1/n_c) \cdot (d_C{}^* - d_C)$$

gilt, wobei $d_C$ bzw. $d_C{}^*$ die Dicke der Hornhaut (5, 5*) vor bzw. nach Entfernung des Volumens (18) bezeichnet und der Radius $R_{CV}{}^*$ iterativ berechenbar ist, indem bei jedem Iterationsschritt aus der Differenz $(R_{CV}{}^* - R_{CV})$ auf eine Dickenänderung $(d_C{}^* - d_C)$ geschlossen wird und das entsprechende daraus erhaltene Ergebnis für die Dickenänderung bei der Berechnung von $R_{CV}{}^*$ im nächsten Iterationsschritt angewendet wird.

5. Vorrichtung oder Verfahren nach Anspruch 1, **dadurch gekennzeichnet, daß** der erste Abschnitt senkrecht zur anterioren Fläche (19) gemessen eine Höhe $d_R$ von mehr als 5 $\mu$m, insbesondere von mindestens 10 $\mu$m hat.

6. Vorrichtung oder Verfahren nach Anspruch 1 oder 5, **dadurch gekennzeichnet, daß** der zweite Abschnitt (26) zur Richtung der Sehachse (OA) in einem Winkel von 80° bis 100° liegt.

7. Vorrichtung oder Verfahren nach Anspruch 6, **dadurch gekennzeichnet, daß** der zweite Abschnitt (26) konkav ist, bezogen auf den Durchtrittspunkt der Sehachse (OA) durch die Hornhautvorderfläche (15).

**Claims**

1. A treatment apparatus for surgical correction of hyperopia in the eye (3), the treatment apparatus (1) having a laser device (L) which is controlled by a control device (12) and separates corneal tissue by applying laser radiation (2), the control device (12) being embodied to control the laser device (L) for emitting the laser radiation (2) into the cornea (5) in such a way that a lenticular volume (18) is isolated in the cornea (5), the removal of which volume from the cornea (18) effects the desired correction of hyperopia, wherein the control device (12) is set up, when controlling the laser device (L), to define the lenticular volume (18) to have a posterior face (20) and an anterior face (19), the latter being curved to a lesser extent at least in a main plane (A-A) than the posterior face (20), and the edges of which are connected via an annular edge face (24), wherein an intersection curve from the edge face (24) and a plane, in which the axis of vision (OA) is contained, has a width (B) transverse to the axis of vision (OA) which is greater than that which would have in the same projection a straight line which is perpendicular at the edge of the posterior or the anterior face (20, 19) on the respective face and connects the anterior face (19) to the posterior face (20), **characterised in that** the edge face (24) has a first portion (25) which discharges perpendicularly into the anterior face (19) and a second portion (26) with a greater inclination with respect to the axis of vision (OA).

2. A method for generating control data for a laser device (L) of a treatment apparatus (1) for surgical correction of hyperopia in the eye (3), which laser device separates corneal tissue by applying laser radiation (2), wherein the control data controls the laser device (L) in operation for emitting the laser radiation (2) into the cornea (5) such that a lenticular volume (18) is isolated in the cornea (5), the removal of which volume from the cornea (18) effects the desired correction of hyperopia, wherein the control data define the lenticular volume (18) to have a posterior face (20) and an anterior face (19), wherein the latter is curved to a lesser extent at least in a main plane (A-A) than the posterior face (20), and the edges of which are connected via an annular edge face (24), wherein an intersection curve from the edge face (24) and a plane, in which the axis of vision (OA) is contained, has a width (B) transverse to the axis of vision (OA) which is greater than that which would have in the same projection a straight line which is perpendicular at the edge of the posterior or the anterior face (20, 19) on the respective face and connects the anterior face (19) to the posterior face (20), **characterised in that** the edge face (24) has a first portion (25) which discharges perpendicularly into the anterior face (19) and a second portion (26) with a greater inclination with respect to the axis of vision (OA).

3. The apparatus or the method according to any one of the above claims, **characterised in that** the anterior face (19) is positioned at a constant distance $d_F$ from the front face of the cornea (15) and the posterior face is curved and has a radius of curvature $R_L = R_{CV}{}^* - d_F$, wherein the following equation is used in determining the control data:

$$R_{CV}{}^* = 1/ (\ (1/R_{CV}) + B_{BR}\ 1(\ (n_C{-}1) \bullet (1 - d_{HS} \cdot B_{BR}))) + F,$$

and $R_{CV}$ is the radius of curvature of the cornea (5) before removal of the volume (18), $n_C$ is the refractive power of a material of the cornea (5), F is a correction factor, $B_{BR}$ is the refractive power of a pair of spectacles (17) suitable for correcting defective vision, and $d_{HS}$ is the distance at which the pair of spectacles (17) having refractive power $B_{BR}$ would have to be positioned before the corneal vertex in order to achieve the desired correction of defective vision by means of the pair of spectacles (17).

4.  The apparatus or the method according to Claim 3, **characterised in that** the following applies

$$F = (1 - 1/n_C) \bullet (d_C^* - d_C),$$

wherein $d_C$ and $d_C^*$ respectively denote the thickness of the cornea (5, 5*) before and after removal of the volume (18) and radius $R_{CV}^*$ can be calculated iteratively **in that** during each iteration step a change in thickness ($d_C^* - d_C$) is concluded from the difference ($R_{CV}^* - R_{CV}$) and the corresponding result obtained therefrom for the change in thickness is applied in the calculation of $R_{CV}^*$ in the next iteration step.

5.  The apparatus or the method according to Claim 1, **characterised in that** the first portion has a height $d_R$ of more than 5 $\mu$m, in particular of at least 10 $\mu$m, measured perpendicular to the anterior face (19).

6.  The apparatus or the method according to Claim 1 or 5, **characterised in that** the second portion (26) is positioned at an angle of from 80° to 100° relative to the direction of the axis of vision (OA).

7.  The apparatus or the method according to Claim 6, **characterised in that** the second portion (26) is concave, based on the point at which the axis of vision (OA) passes through the front face of the cornea (15).

**Revendications**

1.  Dispositif de traitement permettant la correction chirurgicale de l'hyperopie d'un oeil (3), le dispositif de traitement (1) présentant un dispositif laser (L) commandé par un dispositif de commande (12) qui découpe le tissu de la cornée par projection d'un rayon laser (2), dans lequel le dispositif de commande (12) est conçu pour commander le dispositif laser (L) aux fins de l'émission du rayon laser (2) dans la cornée (5) de telle sorte qu'un volume en forme de lenticule (18) est isolé dans la cornée (5), dont l'enlèvement de la cornée (18) a pour effet la correction souhaitée de l'hyperopie, le dispositif de commande (12) étant réalisé pour définir le volume en forme de lenticule (18), lors de la commande du dispositif laser (L), de telle sorte qu'il présente une surface postérieure (20) et une surface antérieure (19), cette dernière étant courbée plus faiblement dans un plan principal (A-A) que la surface postérieure (20) et ses bords étant reliés par une surface de bord annulaire (24), étant entendu qu'une courbe de coupe à partir de la surface de bord (24) et d'un plan dans lequel l'axe de vision (OA) est inclus présente, transversalement par rapport à l'axe de vision (OA), une largeur (B) qui est supérieure à celle qui présente une forme droite, dans le même plan, qui s'étend verticalement sur le bord de la surface postérieure ou antérieure (20, 19) sur la surface respective et relie la surface antérieure (19) avec la surface postérieure (20), **caractérisé en ce que** la surface de bord (24) présente une première section (25) débouchant perpendiculairement dans la surface antérieure (19) et une deuxième section (26) inclinée plus fortement en direction de l'axe de vision (OA).

2.  Procédé de génération de données de commande pour un dispositif laser (L) d'un dispositif de traitement (1) permettant la correction chirurgicale de l'hyperopie d'un oeil (3) qui découpe le tissu de la cornée par projection d'un rayon laser (2), dans lequel au cours du fonctionnement, les données de commande commandent le dispositif laser (L) aux fins de l'émission du rayon laser (2) dans la cornée (5) de telle sorte qu'un volume en forme de lenticule (18) est ainsi isolé dans la cornée (5), dont l'enlèvement de la cornée (18) a pour effet la correction souhaitée de l'hyperopie, dans lequel les données de commande définissent le volume en forme de lenticule (18) de telle sorte qu'il présente une surface postérieure (20) et une surface antérieure (19), cette dernière étant courbée plus faiblement dans un plan principal (A-A) que la surface postérieure (20) et ses bords étant reliés par une surface de bord annulaire (24), étant entendu qu'une courbe de coupe à partir de la surface de bord (24) et d'un plan dans lequel l'axe de vision (OA) est inclus présente, transversalement par rapport à l'axe de vision (OA), une largeur (B) qui est supérieure à celle qui présente une forme droite, dans le même plan, qui s'étend verticalement sur le bord de la surface postérieure ou antérieure (20, 19) sur la surface respective et relie la surface antérieure (19) avec la surface postérieure (20), **caractérisé en ce que** la surface de bord (24) présente une première section (25) débouchant perpendiculairement dans la surface antérieure et une deuxième section (26) inclinée plus fortement en direction de l'axe de vision (OA).

3.  Dispositif ou procédé selon l'une des revendications précédentes, **caractérisé en ce que** la surface antérieure (19) se situe à une distance constante $d_F$ de la surface avant de la cornée (15) et la surface postérieure est courbée et présente un rayon de courbure $R_L = R_{CV}^* - d_F$, l'équation suivante étant utilisée dans la détermination des données de commande :

$$R_{CV}{}^* = 1 \, / \, (\, (1/R_{CV}) + B_{BR} \, / \, (\, (n_c{-}1)\bullet(1 - d_{HS}\bullet B_{BR}))) + F$$

où Rcv est le rayon de courbure de la cornée (5) avant l'enlèvement du volume (18), $n_c$ la vergence du matériau de la cornée (5), F un facteur de correction, $B_{BR}$ la vergence de lunettes adaptées à la correction de l'amétropie (17) et $d_{HS}$ la distance à laquelle les lunettes (17) ayant la vergence $B_{BR}$ devraient se trouver devant le sommet de la cornée pour atteindre la correction souhaitée de l'amétropie au moyen des lunettes (17).

4. Dispositif ou procédé selon la revendication 3, **caractérisé en ce que** l'équation suivante est applicable :

$$F = (1 - 1/n_c) \bullet (d_c{}^* - d_c)$$

où $d_c$ et $d_c{}^*$ désignent respectivement l'épaisseur de la cornée (5, 5*) avant et après l'enlèvement du volume (18) et le rayon $R_{CV}{}^*$ peut être calculé par itération **en ce qu'**à chaque étape d'itération, on conclut à une modification de l'épaisseur ($d_c{}^* - d_c$) à partir de la différence ($R_{CV}{}^* - Rcv$) et le résultat ainsi obtenu est appliqué pour la modification de l'épaisseur dans le calcul de $R_{CV}{}^*$ à l'étape d'itération suivante.

5. Dispositif ou procédé selon la revendication 1, **caractérisé en ce que** la première section, mesurée perpendiculairement à la surface antérieure (19), présente une hauteur $d_R$ de plus de 5 $\mu$m, et en particulier d'au moins 10 $\mu$m.

6. Dispositif ou procédé selon la revendication 1 ou 5, **caractérisé en ce que** la deuxième section (26) se situe dans un angle de 80° à 100° par rapport à la direction de l'axe de vision (OA).

7. Dispositif ou procédé selon la revendication 6, **caractérisé en ce que** la deuxième section (26) est concave par rapport au point de passage de l'axe de vision (OA) à travers la surface avant de la cornée (15).

FIG 1

FIG 2

Fig. 5

Fig. 6

Fig. 1a

FIG 3

$d_F$

$d_L$

$h_F$

$h_L$

Fig. 7

Fig.4

a)

15 5 16 13

$R_{cv}$

$z=0$

3

14

F

z

b)

17 5 13

$d_{HS}$

14

F* F

z

$z=0$

c)

$z=0$ 5

15 13

$R_{cv}$

$R_{cv}^{*}$

5*

15* $z=0$

14

F* F

z

FIG 8

5

21

19 15

23

22 6 20

18

_Fig. 12 b_

_Fig. 12 a_

_Fig. 12 c_

_Fig. 12 d_

Fig. 11a

Fig. 11b

Fig. 10a

Fig. 10b

EP 2 211 803 B1

Fig. 9a

Fig. 9b

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6325792 B1
- US 20060155265 A1
- US 20030212387 A1
- US 5984916 A
- WO 2008125173 A2
- EP 1159986 A1
- US 5549632 A
- DE 69500997 T2
- WO 2004032810 A2
- WO 2005011546 A
- WO 2005011545 A